(19) Europäisches Patentamt
European Patent Office
Office européen des brevets

(11) Publication number: **0 050 407**
**A1**

(12) **EUROPEAN PATENT APPLICATION**

(21) Application number: **81303931.0**

(22) Date of filing: **27.08.81**

(51) Int. Cl.³: **C 07 D 249/08,** C 07 D 403/12, A 61 K 31/41 // C07D295/08, C07C119/18

(30) Priority: **27.08.80 GB 8027744**

(43) Date of publication of application: **28.04.82 Bulletin 82/17**

(84) Designated Contracting States: **AT BE CH DE FR GB IT LI LU NL SE**

(71) Applicant: **GLAXO GROUP LIMITED, Clarges House 6-12 Clarges Street, London W1Y 8DH (GB)**

(72) Inventor: **Bays, David Edmund, 9 Windmill Field, Ware Hertfordshire (GB)**
Inventor: **Carey, Linda, 3 The Lawns Close Melbourn, Royston Hertfordshire (GB)**
Inventor: **Hayes, Roger, 5 Sandringham Road, Potters Bar Hertfordshire (GB)**

(74) Representative: **Marchant, James Ian et al, Elkington and Fife High Holborn House 52/54 High Holborn, London WC1V 6SH (GB)**

(54) **3,5-Disubstituted-1,2,4-triazole compounds, processes for their preparation and pharmaceutical compositions containing them.**

(57) The invention provides compounds of the general formula (I)

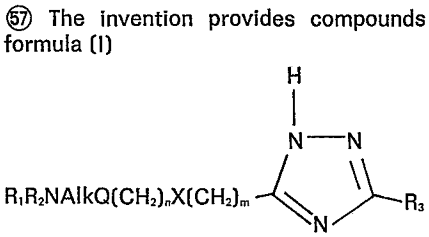

(I)

and physiologically acceptable salts, hydrates and bioprecursors thereof in which

$R_1$ represents hydrogen, $C_{1-10}$ alkyl, cycloalkyl, alkenyl, alkynyl, aralkyl, heteroaralkyl, trifluoroalkyl or $C_{1-10}$ alkyl substituted by hydroxy, alkoxy or dialkylamino; or $C_{1-6}$ alkyl substituted by cycloalkyl;

$R_2$ represents hydrogen or a $C_{1-4}$ alkyl group;

or $R_1$ and $R_2$ may together with the nitrogen atom to which they are attached form a 5-10 membered ring which may be saturated or may contain at least one double bond, may be unsubstituted or substituted by one or more $C_{1-3}$ alkyl groups or a hydroxy group and/or may contain another heteroatom, selected from oxygen or sulphur;

Alk represents a straight or branched $C_{1-6}$ alkylene chain;

Q represents a benzene ring in which incorporation into the rest of the molecule is through bonds at the 1- and 3-positions or 1- and 4-positions;

X represents oxygen, sulphur or a methylene group;

n represents zero, 1 or 2;

m represents 2, 3 or 4 with the proviso that n + m is at least 3;

$R_3$ represents alkenyl, alkoxy or alkyl substituted by hydroxy, acyloxy, alkoxy, amino, alkylamino, dialkylamino, aryl, heteroaryl or $C_{1-4}$ alkylsulphonyl; or $R_3$ represents $COR_4$ or $CR_4{=}NOH$ where $R_4$ represents hydrogen or $C_{1-4}$ alkyl.

The compounds show pharmacological activity as selective histamine $H_2$-antagonists.

EP 0 050 407 A1

This invention relates to novel heterocyclic derivatives having action on histamine receptors, to processes for the preparation thereof, to pharmaceutical compositions containing them and to their use in therapeutics.

Certain novel heterocyclic derivatives have now been found which have potent activity as $H_2$-antagonists. These compounds, which are more particularly described below, for example show inhibition of the secretion of gastric acid when this is stimulated via histamine receptors (Ash and Schild, Brit. J. Pharmacol. Chemother, 1966, 27, 427). Their ability to do so can be demonstrated in the perfused rat stomach using the method described in German Offenlegungsschrift No. 2,734,070, modified by the use of sodium pentobarbitone (50 mg/kg) as anaesthetic instead of urethane, and in conscious dogs equipped with Heidenhain pouches using the method described by Black et al, Nature 1972, 236, 385. Furthermore, the compounds antagonise the effect of histamine on the contraction frequency of isolated guinea pig right atrium but do not modify histamine induced contractions of isolated gastrointestinal smooth muscle which are mediated via $H_1$-receptors.

Compounds with histamine $H_2$-blocking activity may be used in the treatment of conditions where there is an advantage in lowering gastric acidity, particularly in gastric and peptic ulceration, as a prophylactic measure in surgical procedures, and in the treatment of allergic and inflammatory conditions where histamine is a known mediator. Thus they may be used for example, either alone, or in combination with other active ingredients in the treatment of allergic and inflammatory conditions of the skin.

The present invention provides compounds of the general formula (I)

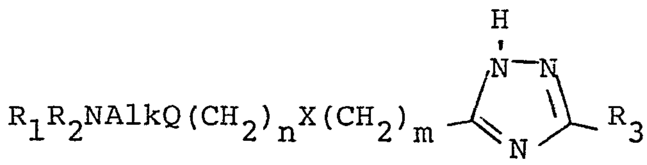

(I)

and physiologically acceptable salts, hydrates and bioprecursors thereof in which

$R_1$ represents hydrogen, $C_{1-10}$ alkyl, cycloalkyl, alkenyl, alkynyl, aralkyl, heteroaralkyl, trifluoroalkyl or $C_{1-10}$ alkyl substituted by hydroxy, alkoxy or dialkylamino; or $C_{1-6}$ alkyl substituted by cycloalkyl;

$R_2$ represents hydrogen or a $C_{1-4}$ alkyl group;

or $R_1$ and $R_2$ may together with the nitrogen atom to which they are attached form a 5-10 membered ring which may be saturated or may contain at least one double bond, may be unsubstituted or substituted by one or more $C_{1-3}$ alkyl groups or a hydroxy group and/or may contain another heteroatom selected from oxygen or sulphur;

Alk represents a straight or branched $C_{1-6}$ alkylene chain;

Q represents a benzene ring in which incorporation into the rest of the molecule is through bonds at the 1- and 3-positions or 1- and 4-positions;

X represents oxygen, sulphur or a methylene group;

n represents zero, 1 or 2;

m represents 2, 3 or 4; with the proviso that n + m is at least 3;

$R_3$ represents alkenyl, alkoxy or alkyl substituted by hydroxy, acyloxy, alkoxy, amino, alkylamino, dialkylamino, aryl, heteroaryl or $C_{1-4}$ alkylsulphonyl; or $R_3$ represents $COR_4$ or $CR_4$=NOH where $R_4$ represents hydrogen or $C_{1-4}$ alkyl.

The term 'alkyl' as a group or part of a group means that the group is straight or branched and unless otherwise stated has preferably 1 to 6 carbon atoms, more preferably

1 to 4 carbon atoms, e.g. methyl or ethyl, and the terms 'alkenyl' and 'alkynyl' mean that the group is straight or branched and has 3 to 6 carbon atoms. The term 'cycloalkyl' means that the group has 3 to 8 carbon atoms. The acyl portion of an acyloxyalkyl group means an aroyl, aralkanoyl or $C_{1-6}$ alkanoyl group. Examples of acyloxyalkyl groups include acetoxymethyl, formyloxymethyl, benzoyloxymethyl and phenylacetoxymethyl. The term 'aryl' as a group or part of a group preferably represents phenyl or substituted phenyl, for example phenyl substituted with one or more $C_{1-3}$ alkyl, hydroxy, or $C_{1-3}$ alkoxy groups or halogen atoms. The term heteroaryl as a group or part of a heteroaralkyl group preferably refers to a monocyclic unsaturated 5 or 6-membered ring containing an oxygen, nitrogen or sulphur atom, e.g. furyl, pyridyl or thienyl. The alkyl part of the heteroaralkyl group is a straight or branched $C_{1-4}$ alkylene chain e.g. methylene.

When the group $R_1R_2N$ represents a heterocyclic ring it preferably contains 5-8 members and optionally contains one double bond and/or is substituted by one or two $C_{1-3}$ alkyl (e.g. methyl) groups or a hydroxy group (e.g. pyrrolidino, piperidino, hexamethylenimino, heptamethylenimino, tetrahydropyridino, 4-hydroxypiperidino, 2,6-dimethylmorpholino or thiamorpholino).

Preferred groupings for the compounds of the invention are as follows:

Q is a benzene ring incorporated into the rest of the molecule through bonds at the 1- and 3-positions;

X is oxygen, n is zero and m is 3 or 4;

$R_1R_2N$ is a 5-7 membered ring, e.g. hexamethylenimino or, more preferably pyrrolidino or piperidino;

Alk is a $C_{1-4}$ alkylene chain more preferably methylene;

$R_3$ is aralkyl (e.g. benzyl) or, more preferably, hydroxy $C_{1-4}$ alkyl (e.g. hydroxymethyl or

hydroxyethyl), acyloxy $C_{1-4}$ alkyl (e.g. acyloxy $C_{1-2}$ alkyl such as acetoxymethyl or benzoyloxymethyl), formyl, the group CH=NOH or methylsulphonylmethyl.

Particularly preferred compounds are:

5-[4-[3-(1-piperidinylmethyl)phenoxy]butyl]-1H-1,2,4-triazole-3-methanol,

5-[4-[3-(1-piperidinylmethyl)phenoxy]butyl]-1H-1,2,4-triazole-3-(2-ethanol),

5-[4-[3-(1-piperidinylmethyl)phenoxy]propyl]-1H-1,2,4-triazole-3-methanol,

5-[4-[3-(1-piperidinylmethyl)phenoxy]butyl]-1H-1,2,4-triazole-3-methylacetate,

3-(methylsulphonyl)methyl-5-[4-[3-(1-piperidinylmethyl)phenoxy]butyl]-1H-1,2,4-triazole,

5-[4-[3-(1-piperidinylmethyl)phenoxy]butyl]-1H-1,2,4-triazole-3-carboxaldehyde hydrate, and

5-[4-[3-(1-piperidinylmethyl)phenoxy]butyl]-1H-1,2,4-triazole-3-carboxyaldehyde oxime,

and their physiologically accepatble salts.

The invention includes the compounds of formula (I) in the form of physiologically acceptable salts with inorganic and organic acids. Particularly useful salts include hydrochlorides, hydrobromides and sulphates, methanesulphonates, acetates, maleates, succinates, tartrates, benzoates, citrates and fumarates. The compounds of formula (I) and their salts may also form hydrates, which hydrates are also to be considered as part of the invention. The compounds of formula (I) can exhibit tautomerism and the formula is intended to cover all tautomers. Where optical isomers may exist the formula is intended to cover all diastereoisomers and optical enantiomers. The term bioprecursors as used herein means compounds which have a structure different to that of the compounds of formula (I) but which, upon administration to the animal or human being, are converted in the body into a compound

of formula (I).

The compounds according to the invention, preferably in the form of a salt, may be formulated for administration in any convenient way and the invention includes within its scope pharmaceutical compositions containing at least one compound according to the invention adapted for use in human or veterinary medicine. Such compositions may be formulated in a conventional manner using one or more pharmaceutically accetable carriers or excipients. Such compositions may also contain if required other active ingredients, e.g. $H_1$-antagonists.

Thus the compounds according to the invention may be formulated for oral, buccal, topical, parenteral or rectal administration. Oral administration is preferred.

For oral administration, the pharmaceutical composition may take the form of for example, tablets, capsules, powders, solutions, syrups or suspensions prepared by conventional means with acceptable excipients. For buccal administration the composition may take the form of tablets or lozenges formulated in conventional manner.

The compounds of the invention may be formulated for parenteral administration by bolus injection or continuous infusion. Formulations for injection may be presented in unit dosage form in ampoules, or in multidose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water before use.

The compounds of the invention may also be formulated in rectal compositions such as suppositories or retention enemas, e.g. containing conventional suppository bases such as cocoa butter or other glyceride.

For topical application, the compounds of the invention may be formulated as ointments, creams, gels, lotions, powders or sprays in a conventional manner.

For internal administration a convenient daily dosage regime of the compounds according to the invention is 1 to 4 doses to the total of 100 mg to 2 g per day, preferably 500 mg to 1 g per day, dependent upon the condition of the patient.

It will be appreciated in the the methods for the preparation of the compound of formula (I) given below that for certain reaction steps it may be necessary to protect various reactive substituents in the starting materials for a particular reaction and subsequently to remove the protecting group. Such protection and subsequent deprotection may be particularly pertinent when $R_1$ and/or $R_2$ are hydrogen and/or when the substituent $R_3$ is an alkyl group bearing a hydroxyl group or a primary or secondary amine. Standard protection and deprotection procedures can be employed, for example, amines may be protected by formation of a phthalimide group which may subsequently be cleaved by treatment with a hydrazine e.g. hydrazine hydrate or a primary amine for example methylamine.

In describing the processes which may be used for preparing the compounds of formula (I) or intermediates useful in the preparation thereof any of the groups $R_1$, $R_2$, $R_3$, Alk, Q, X, n and m are as defined in formula (I) unless otherwise stated.

Compounds of formula (I) may be prepared by cyclisation of the compound of formula (II) in which one of V and Y represents NH and the other represents oxygen, sulphur or NH. It should be noted that when V or Y is sulphur, tautomerisation with the adjacent NH is possible [-N=C(SH)-], and the resulting thiol may be converted into the corresponding S-alkyl, e.g. S-methyl, derivative which may also be used in the cyclisation.

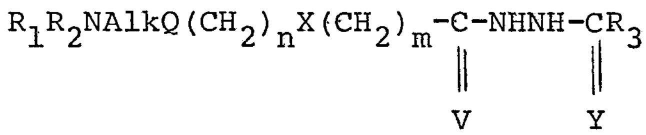 (II)

Thus in one embodiment of the cyclisation process compounds of formula (I) may be prepared by heating a compound of formula (II) in which V is oxygen and Y is NH or V is NH and Y is oxygen. The reaction is preferably carried out in the absence or, more preferably, in the presence of a solvent e.g. ethanol at an elevated temperature e.g. 60-160°C.

The compounds of formula (II) wherein V is NH and Y is O or V is O and Y is NH may be prepared by reaction of an iminoether (III) or (IV) with a hydrazide (V) or (VI) respectively in the presence of a solvent e.g. methanol or ethanol. Preferably the reaction is carried out at 0-25°C.

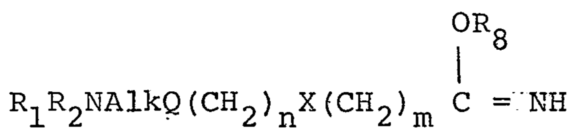 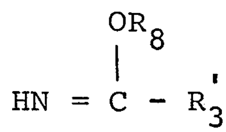

(III) (IV)

where $R_3'$ is the group $R_3$ or a group convertible thereto such as haloalkyl which may be converted into an acyloxy-alkyl group; $R_8$ is an alkyl group e.g. methyl or ethyl.

$NH_2NHCOR_3'$ (V)

$R_1R_2NAlkQ(CH_2)_nX(CH_2)_mCONHNH_2$ (VI)

The iminoethers (III) and (IV) may be prepared from the corresponding nitriles (VII) or $R_3'CN$ by treatment with an anhydrous alcohol e.g. ethanol in the presence of hydrogen chloride preferably at a low temperature e.g. 0-3°C. The reaction is preferably carried out in the presence of a solvent e.g. methylene chloride.

$R_1R_2NAlkQ(CH_2)_nX(CH_2)_mCN$ (VII)

The hydrazides (V) and (VI) may be prepared by reaction of the esters (VIII) and $R_3'CO_2R_9$ with hydrazine hydrate at 25-100°C in the absence or presence of a solvent e.g. ethanol.

$$R_1R_2NAlkQ(CH_2)_nX(CH_2)_mCO_2R_9 \qquad (VIII)$$

where $R_9 = C_{1-4}$ alkyl.

The nitriles (VII) and the esters (VIII) may be prepared by using methods analogous to those described in British Patent Specification No. 2047238A.

Aldehydes or ketones of formula (I) in which $R_3$ is the group $-COR_4$ may be prepared by oxidising the corresponding hydroxyalkyl compound in which $R_3$ is $-CHR_4OH$ using for example oxalyl chloride and dimethylsulphoxide, or activated manganese dioxide in a solvent such as chloroform.

Compounds of formula (I) in which $R_3$ is the group $-CR_4{=}NOH$ may be prepared by reacting the corresponding carbonyl compound ($R_3$ is $-COR_4$) with hydroxylamine in a suitable solvent such as ethanol, optionally with heating.

Compounds of formula (I) in which $R_3$ represents an acyloxyalkyl group may be prepared by reacting a compound of formula (I) in which $R_3$ represents a hydroxyalkyl group with a carboxylic acid or an activated derivative thereof which corresponds to the acyl group. Suitable activated derivatives include acid halides, e.g. acid chlorides and acid anhydrides. When an acid chloride is used, the reaction is preferably carried out in for example pyridine at room temperature.

Where the product of any of the above processes is a free base and a salt is required, the salt may be formed in a conventional manner. Thus for example, a generally convenient method of forming the salts is to mix appropriate quantities of the free base and the acid in an appropriate solvent(s) e.g. an alcohol such

as ethanol or an ester such as ethyl acetate.

The invention is illustrated but not limited by the following Examples and Preparations. All temperatures are in °C.

Preparation 1

(a) 5-[3-(1-Piperidinylmethyl)phenoxy]pentanenitrile

Sodium hydride (2.4 g) and 1-(3-hydroxyphenyl)methylpiperidine (19.1 g) in dry dimethylformamide were stirred under a nitrogen atmosphere at room temperature for 5 h. 5-Bromovaleronitrile (16.2 g) was added, the mixture was stirred for 12 h, diluted with water, and extracted with ether. Distillation of the organic extracts gave the title compound as a yellow oil (23.7 g) b.p. 200°/0.1 mm.

(b) Similarly prepared from sodium hydride (5.4 g), 3-hydroxy-benzaldehyde (20 g), and 5-bromovaleronitrile (26.6 g) was 5-(3-formylphenoxy)pentanenitrile (23.8 g) b.p. 190-194° (0.1 mm)

(c) Similarly prepared from sodium hydride (0.87 g), 1-(3-hydroxyphenyl)methyldimethylamine (4.0 g) and 5-bromovaleronitrile (4.29 g) was 5-[3-[(dimethylamino)-methyl]phenoxy]pentanenitrile (5.17 g) b.p. 180° (0.05 mm).

Preparation 2

(a) 5-[3-(1-Pyrrolidinylmethyl)phenoxy]pentanenitrile

A solution of 5-(3-formylphenoxy)pentanenitrile (3.5 g) and pyrrolidine (1.34 g) in ethanol (80 ml) was hydrogenated over 10% palladium-carbon, at atmospheric pressure, until the theoretical volume of hydrogen had been consumed. The mixture was filtered and the filtrate evaporated *in vacuo*. The residue was distilled to give the title compound (3.4 g) as a pale yellow oil b.p. 240° (0.1 mm).

(b) Similarly prepared from 5-(3-formylphenoxy)pentane-nitrile (3 g) and hexamethyleneimine (1.61 g) was 5-[3-(1-hexamethyleneiminylmethyl)phenoxy]pentanenitrile except that at the end of the reaction, the residual oil was dissolved in 2N hydrochloric acid and washed with ether. The acidic layer was basified with potassium carbonate and the product extracted into ethyl acetate. The ethyl acetate extract was washed, dried and evaporated and the residue distilled to give the title compound (2.2 g), b.p. 210° (0.8 mm).

Preparation 3

(a) Ethyl 5-[3-[(Dimethylamino)methyl]phenoxy]-pentanimidate

A solution of 5-[3-[(dimethylamino)methyl]phenoxy]pentanenitrile (2.0 g) and dry ethanol (0.59 g) in dry dichloromethane (30 ml) was saturated with gaseous hydrogen chloride at 0° for 1 h, the flask stoppered and stored overnight at 5°. The mixture was diluted with dichloromethane, washed with aqueous sodium bicarbonate and evaporated in vacuo to give the title compound (2.3 g) as an oil.

Nmr ($CDCl_3$) 2.82, t, (1H); 3.1-3.3, m, (3H); 5.98, q, (2H); 6.10, t, (2H); 6.68, s, (2H); 7.8, s, (6H); 8.25, m, (4H); 8.73, t, (3H).

(b) Similarly prepared from 5-[3-(1-pyrrolidinylmethyl) phenoxy]pentanenitrile (3.0 g) and ethanol (0.59 g) was ethyl 5-[3-[(1-pyrrolidinylmethyl)phenoxy]pentanimidate (2.1 g).

Ir (liquid film): 3325, 2830, 2785, 1650 $cm^{-1}$.

(c) Similarly prepared from 5-[3-(1-hexamethyleneiminylmethyl)phenoxy]pentanenitrile (2.0 g) and ethanol (0.41 g) was ethyl 5-[3-[(1-hexamethyleneiminylmethyl)phenoxy] pentanimidate (2.2 g).

Ir (liquid film): 3325, 2810, 2760, 1650 $cm^{-1}$.

(d) Similarly prepared from 4-[3-(1-piperidinylmethyl) phenoxy]butanenitrile (2.0 g) and ethanol (0.53 g) was ethyl 4-[3-(1-piperidinylmethyl)phenoxy]butanimidate (2.3 g)

Ir (liquid film): 3325, 2800, 2760, 1650 $cm^{-1}$.

Preparation 4

Ethyl 5-[3-(1-piperidinylmethyl)phenoxy]pentanimidate

A solution of 5-[3-(1-piperidinylmethyl)phenoxy]pentanenitrile (1.66 g) in dry methylene chloride (30 ml) and dry ethanol (0.39 ml) was saturated with hydrogen chloride gas at 0° for 3 h. The mixture was stirred at 0° for 20 h, diluted with methylene chloride and washed with sodium bicarbonate solution. The organic extract was evaporated in vacuo to

yield the title compound as an oil (1.9 g)
T.l.c. silica:methanol Rf 0.4

EXAMPLE 1

5-[3-[3-(1-Piperidinylmethyl)phenoxy]propyl]-1H-1,2,4-triazole-3-methanol

A solution of ethyl 4-[3-(1-piperidinylmethyl)phenoxy]-butanimidate (3.43 g) and glycolic acid hydrazide (1.02 g) in absolute ethanol (60 ml) was stirred at room temperature for 18 h and then heated under reflux for 7 h. The solvent was removed *in vacuo* and the residue was partitioned between chloroform and water. The organic phase was evaporated *in vacuo* and the residue was triturated with ether to give the title compound (1.4 g) as colourless crystals m.p. 106-107°, recrystallised from ethyl acetate.

Assay Found : C, 65.3; H, 7.9; N, 16.7;
$C_{18}H_{26}N_4O_2$ requires : C, 65.4; H, 7.9; N, 17.0%.

(b) Similarly prepared from ethyl 5-[3-[(dimethylamino)-methyl]phenoxy]pentanimidate (2.3 g) and glycolic acid hydrazide (0.72 g) was 5-[4-[3-[(dimethylamino)methyl]phenoxy]butyl]-1H-1,2,4-triazole-3-methanol citrate (1:2) except that the reaction mixture was heated under reflux for 3 h. After work-up, trituration with ether gave an oil (1.8 g). An analytical sample (0.4 g) was dissolved in ethyl acetate and treated with an excess of citric acid in ethyl acetate to give the title compound (0.18 g) as a white powder m.p. 61-63°.

NMR ($d^6$-DMSO): 2.5, m, (1H); 2.8-3.0, m, (3H); 5.33, s, (2H); 5.70, s, (2H); 5.90, m, (2H); 7.10, s, (6H); 7.15, m, (6H); 7.9-8.3, m, (4H).

EXAMPLE 2

5-[4-[3-(1-Piperidinylmethyl)phenoxy]butyl]-1H-1,2,4-triazole-3-(2-ethanol) maleate (1:2)

A solution of ethyl 5-[3-(1-piperidinylmethyl)phenoxy] pentanimidate (5.7 g) and 3-hydroxypropionic acid hydrazide (2.52 g) in ethanol was stirred at 20° for 18 h and then heated under reflux for 3 h. The solvent was removed *in vacuo*, and the residue was partitioned between chloroform and water. The organic extracts were evaporated and the oil chromatographed on silica using dichloromethane:ethanol: ammonia 50:8:1. The oil was dissolved in ethanol-ethyl acetate and treated with an excess of maleic acid to give a white solid which was recrystallised from ethanol-ethyl acetate to give the title compound (2.75 g) as white crystals

m.p. 97-98°.

Found : C, 56.8; H, 6.6; N, 9.5;

$C_{20}H_{30}N_4O_2 \cdot C_8H_8O_8$ requires : C, 56.9; H, 6.5; N, 9.5%.

(b) Similarly prepared from ethyl 5-[3-(1-pyrrolidinylmethyl)phenoxy]pentanimidate (2.1 g) and glycolic acid hydrazide (0.684 g) was 5-[4-[3-(1-pyrrolidinylmethyl)phenoxy]butyl]-1H-1,2,4-triazole-3-methanol maleate (1:2) except that the reaction mixture was heated under reflux for 6 h. The crude product was chromatographed on silica using methanol:0.88 ammonia 79:1 to give an oil. This oil was dissolved in ethyl acetate and treated with an excess of maleic acid in ethyl acetate to give the <u>title compound</u> (0.24 g) as a white powder m.p. 114-116°.

Found : C, 55.2; H, 6.0; N, 9.8;

$C_{18}H_{26}N_4O_2 \cdot C_8H_8O_8$ requires : C, 55.5; H, 6.1; N, 9.9%.

(c) Similarly prepared from ethyl 5-[3-(1-hexamethyleneiminylmethyl)phenoxy]pentanimidate (2.51 g), and glycolic acid hydrazide (0.67 g) was 5-[4-[3-(1-hexamethyleneiminylmethyl)phenoxy]butyl]-1H-1,2,4-triazole-3-methanoloxalate (1:2) except that the reaction mixture was heated under reflux for 4 h. The crude product was chromatographed on silica using chloroform→ chloroform:methanol 9:1 to yield an oil. The oil was dissolved in ethyl acetate and treated with an excess of oxalic acid in ethanol to give the <u>title compound</u> (0.11 g) as a white powder m.p. 150-151°

Found : C, 53.3; H, 6.4; N, 10.0;

$C_{20}H_{30}N_4O_2 \cdot C_4H_4O_8$ requires : C, 53.5; H, 6.4; N, 10.4%.

(d) Similarly prepared from ethyl 5-[3-(1-piperidinylmethyl)phenoxy]pentanimidate (3.5 g) and 4-hydroxybutanoic acid hydrazide (1.4 g) was 5-[4-[3-(1-piperidinylmethyl)phenoxy]butyl]-1H-1,2,4-triazole-3-(3-propanol)maleate (1:2) (3.4 g) m.p. 97-98°.

Found : C, 57.4; H, 6.7; N, 9.1;

$C_{21}H_{32}N_4O_2 \cdot C_8H_8O_8$ requires : C, 57.6; N, 6.7; N, 9.3%.

(e) Similarly prepared from ethyl 5-[3-(1-piperidinylmethyl)phenoxy]pentanimidate (3.5 g) and lactic acid hydrazide (1.26 g) was 5-[4-[3-(1-piperidinylmethyl)phenoxy]

butyl]-1H-1,2,4-triazole-3-(1-ethanol)oxalate (1:2) except that the base was treated with an excess of oxalic acid in ethanol to give the title compound (0.6 g) m.p. 154-156°.

Found : C, 53.2; H, 6.3; N, 10.2;

$C_{20}H_{30}N_4O_2.C_4H_4O_8$ requires : C, 53.5; H, 6.4; N, 10.4%.

EXAMPLE 3

5-[4-[3-(1-Piperidinylmethyl)phenoxy]butyl]-1H-1,2,4-triazole-3-methanol acetate(ester)fumarate

A solution of 5-[4-[3-(1-piperidinylmethyl)phenoxy]butyl]-1H-1,2,4-triazole-3-methanol (1.73 g) in glacial acetic acid (20 ml) was heated under reflux for 18 h. The solution was evaporated and the residue dissolved in chloroform and washed successively with aqueous sodium carbonate and water, dried and evaporated. The residue was chromatographed on silica using chloroform:methanol (4:1) to give a gum (1.75 g). This gum was dissolved in methanol containing fumaric acid (0.47 g) and triturated with ether to give the title compound (1.8 g) as a white powder m.p. 74-78°

Found : C, 59.8; H, 7.0; N, 10.9;

$C_{21}H_{30}N_4O_3.C_4H_4O_4$ requires : C, 59.8; H, 6.8; N, 11.1%.

EXAMPLE 4

5-[4-[3-(1-Piperidinylmethyl)phenoxy]butyl]-1H-1,2,4-triazole-3-methanol benzoate(ester)fumarate

A solution of 5-[4-[3-(1-piperidinylmethyl)phenoxy]butyl]-1H-1,2,4-triazole-3-methanol (1.1 g) and benzoyl chloride (0.494 g) in anhydrous pyridine (10 ml) was stirred at room temperature for 4 h. The solution was evaporated and the residue chromatographed on silica, chloroform:methanol (9:1) to give a gum. To this gum was added a solution of fumaric acid (0.132 g) in methanol (2 ml) which on trituration with ether gave the title compound (0.25 g) as a white foam m.p. 55-60°

Nmr ($D_2O$) 1.9, m, (2H); 2.3-3.3, m, (7H); 4.62, s, (2H); 6.07, t, (2H); 6.80, s, (2H); 7.20, t, (2H); 7.60, m, (4H); 7.8-8.7, m, (10H).

EXAMPLE 5

1-[[3-[4-[3-(Phenylmethyl)-1H-1,2,4-triazol-5-yl]butoxy]phenyl]methyl]piperidine

Benzene acetic acid 2-[1-imino-5-[3-(1-piperidinylmethyl)phenoxy]pentyl]hydrazide

A solution of phenylacetic acid hydrazide (1.06 g) in dry ethanol (20 ml) was added dropwise to a solution of ethyl 5-[3-(1-piperidinylmethyl)phenoxy]pentanimidate (1.5 g) in dry ethanol (40 ml) at 5°. The mixture was stirred at 25° for 2 h, filtered, and the filtrate was evaporated under reduced pressure. The residue was recrystallised from ethyl acetate to give the title compound as a white powder (1.3 g) m.p. 143.5-144.5°

1-[[3-[4-[3-(Phenylmethyl)-1H-1,2,4-triazol-5-yl]butoxy]phenyl]methyl]piperidine

Benzeneacetic acid 2-[1-imino-5-[3-(1-piperidinylmethyl)phenoxy]pentyl]hydrazine (0.5 g) was heated at 155° for 10 min. The residual oil was dissolved in hot cyclohexane. The cooled solution was decanted from the precipitated oil, and triturated to give the title compound as a white solid (0.25 g) m.p. 89.5-90.5°.

Assay Found : C, 74.45; H, 7.95; N, 13.75

$C_{25}H_{32}N_4O$ requires : C, 74.25; H, 7.90; N, 13.85%.

EXAMPLE 6

5-[4-[3-(1-Piperidinylmethyl)phenoxy]butyl-1H-1,2,4-triazole-3-methanol

A solution of glycolic acid hydrazide (0.54 g) and ethyl 5-[3-(1-piperidinylmethyl)phenoxy]pentanimidate (1.85 g) in dry ethanol (20 ml) was stirred for 18 h at room temperature. The solvent was removed in vacuo and the residue was partitioned between chloroform and brine. The organic phase was evaporated in vacuo to give a solid (1.94 g) which was heated on a steam-bath for 2 h, without further purification. The residue was triturated with ether to give the title compound as a white solid (0.94 g), m.p. 119-20°

Assay Found : C, 66.1; H, 8.1; N, 16.0

$C_{19}H_{28}N_4O_2$ requires : C, 66.2; H, 8.2; N, 16.2%.

EXAMPLE 7

3-(Methylsulphonyl)methyl-5-[4-[3-(1-piperidinylmethyl)phenoxy]butyl]-1H-1,2,4-triazole

(Methylsulphonyl)acetic Acid Hydrazide

Hydrazine hydrate (0.32 g) was added to a solution of ethyl (methylsulphonyl) acetate (1.0 g) in ethanol (20 ml) and the mixture stirred at 20° during 5 h. The precipitate was collected and dried to give the title compound (0.38 g), crystallised from ethyl acetate, m.p. 83-85°.

3-(Methylsulphonyl)methyl-5-[4-[3-(1-piperidinylmethyl)phenoxy]butyl]-1H-1,2,4-triazole

A solution of ethyl 5-[3-(1-piperidinylmethyl)phenoxy]pentanimidate (3.0 g) and (methylsulphonyl)acetic acid hydrazide (1.43 g) in ethanol (60 ml) was stirred at room temperature for 18 h and then heated under reflux for 7 h. The solvent was removed *in vacuo* and the residue was partitioned between chloroform and brine. The organic phase was evaporated *in vacuo* to give an oil which, after trituration with dry ether, gave a solid. This solid was dissolved in 2N hydrochloric acid and basified (pH 9) with aqueous sodium bicarbonate solution. The aqueous solution was extracted continuously with hexane for 24 h. The aqueous layer was then extracted with ether and the ether extract dried and evaporated to give, after trituration with hexane, the title compound (0.5 g) as colourless crystals m.p. 79-80°.

NMR ($d^6$- DMSO) 2.80, t, (1H); 3.0-3.3, m, (2H); 5.62, s, (2H); 6.06, t, (2H); 6.52, s, (2H); 6.97, s, (3H); 7.20, t, (2H); 7.5-7.7, m, (4H); 7.9-8.28, m, (4H); 8.28-8.8, m, (6H).

## EXAMPLE 8

5-[4-[3-(1-Piperidinylmethyl)phenoxy]butyl]-1H-1,2,4-triazole-3-carboxaldehyde hydrate

A mixture of 5-[4-[3-(1-piperidinylmethyl)phenoxy]butyl]-1H-1,2,4-triazole-3-methanol (0.6 g) and activated manganese dioxide (2.1 g) in chloroform (20 ml) was stirred at room temperature for 24 h. The suspension was filtered and the filtrate evaporated. The residue was chromatographed on silica using chloroform:methanol (8:1) to give the title compound (0.45 g) as a glass. Trituration under hexane gave a white powder m.p. 53-55°.

| | | | |
|---|---|---|---|
| Assay Found: | C, 63.6; | H, 7.6; | N, 15.4; |
| $C_{19}H_{26}N_4O_2.H_2O$ requires : | C, 63.3; | H, 7.8; | N, 15.5% |

EXAMPLE 9

5-[4-[3-(1-Piperidinylmethyl)phenoxy]butyl]-1*H*-1,2,4,-triazole-3-carboxaldehyde oxime

5-[4-[3-(1-Piperidinylmethyl)phenoxy]butyl]-1*H*-1,2,4-triazole-3-carboxaldehyde (0.69 g) was dissolved in a solution of hydroxylamine (0.13 g) in ethanol. The mixture was allowed to stand for 20 h and the resulting prepciptate filtered off. The filtrate was extracted with ethyl acetate and the extracts evaporated to a solid residue. The combined solids were recrystallised from isopropanol to give the title compound (0.26 g) as a white solid m.p. 155-156.5°.

| | | | | |
|---|---|---|---|---|
| Found | : | C, 64.0; | H, 7.6; | N, 19.1; |
| $C_{19}H_{27}N_5O_2$ requires | : | C, 63.8; | H, 7.6; | N, 19.6%. |

Examples of Pharmaceutical Compositions

| Tablets | mg/tablet | mg/tablet |
|---|---|---|
| Active ingredient | 20.0 | 40.0 |
| Microcrystalline cellulose BPC | 99.5 | 199.0 |
| Magnesium stearate B.P. | 0.5 | 1.0 |
| Compression weight | 120.0 | 240.0 |

The drug is sieved through a 250 µm sieve, blended with the excipients and compressed using 6.5 mm and 8.0 mm diameter punches for the 20 and 40 mg strengths respectively. Tablets of other strengths may be prepared by increasing the compression weight and using punches to suit.

The tablets may be film coated with suitable film forming materials, e.g. methyl cellulose, ethyl cellulose or hydroxypropylmethyl cellulose, using standard techniques. Alternatively the tablets may be sugar coated.

Injection for Intravenous Administration

| | % w/v |
|---|---|
| Active ingredient | 0.25 |
| Water for Injections BP To | 100.00 |

Sodium chloride may be added to adjust the tonicity of the solution and the pH may be adjusted to that of maximum stability using dilute acid or alkali or suitable buffer salts.

The solution is prepared, clarified and filled under nitrogen into appropriate sized ampoules sealed by fusion of the glass. The injection is sterilised by heating in an autoclave using one of the acceptable cycles. Alternatively the solution may be sterilised by filtration and filled into sterile ampoules under aseptic conditions.

The compounds of Examples 2(a), 2(e), 3, 6, 7, 8 and 9 have $ED_{50}$ values of less than 0.63 mg/kg in the perfused rat stomach test.

The compounds of the invention have in general low toxicity at doses at which they effectively inhibit gastric acid secretion. Thus for example the compounds of Examples 7 and 9 produced no toxic effects when administered intravenously to rats at doses of at least twice their $ED_{50}$ values. In addition, the compound of Example 6 produced no toxic effects when administered orally to rats at a dose of 400 mg/kg.

CLAIMS

1. Compounds of the general formula (I)

$R_1R_2NAlkQ(CH_2)_nX(CH_2)_m$ 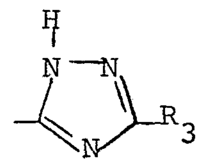 (I)

and physiologically acceptable salts, hydrates and bio-precursors thereof in which

$R_1$ represents hydrogen, $C_{1-10}$ alkyl, cycloalkyl, alkenyl, alkynyl, aralkyl, heteroaralkyl, trifluoroalkyl or $C_{1-10}$ alkyl substituted by hydroxy, alkoxy or dialkylamino; or $C_{1-6}$ alkyl substituted by cycloalkyl;

$R_2$ represents hydrogen or a $C_{1-4}$ alkyl group;

or $R_1$ and $R_2$ may together with the nitrogen atom to which they are attached form a 5-10 membered ring which may be saturated or may contain at least one double bond, may be unsubstituted or substituted by one or more $C_{1-3}$ alkyl groups or a hydroxy group and/or may contain another heteroatom selected from oxygen or sulphur;

Alk represents a straight or branched $C_{1-6}$ alkylene chain;

Q represents a benzene ring in which incorportation into the rest of the molecule is through bonds at the 1- and 3-positions or 1- and 4-positions;

X represents oxygen, sulphur or a methylene group;

n represents zero, 1 or 2;

m represents 2, 3 or 4 with the proviso that n + m is at least 3;

$R_3$ represents alkenyl, alkoxy or alkyl substituted by hydroxy, acyloxy, alkoxy, amino, alkylamino, dialkylamino, aryl, heteroaryl or $C_{1-4}$ alkylsulphonyl; or $R_3$ represents $COR_4$ or $CR_4$=NOH where $R_4$ represents hydrogen or $C_{1-4}$ alkyl.

2. Compounds as claimed in claim 1 in which Q is a benzene ring incoporated into the rest of the molecule through bonds at the 1- and 3-positions.

3. Compounds as claimed in claim 1 or 2 in which X is oxygen, n is zero and m is 3 or 4.

4. Compounds as claimed in any of claims 1 to 3 in which $R_1R_2N$ is a 5-7 membered ring.

5. Compounds as claimed in any of claims 1 to 4 in which Alk is methylene.

6. Compounds as claimed in any of claims 1 to 5 in which $R_3$ represents alkenyl, alkoxy, or alkyl substituted by hydroxy, acyloxy, alkoxy, amino, alkylamino, dialkylamino, aryl or heteroaryl.

7. Compounds as claimed in any of claims 1 to 5 in which $R_3$ is aralkyl, hydroxy $C_{1-4}$ alkyl, acyloxy $C_{1-4}$ alkyl, formyl, the group CH=NOH or methylsulphonylmethyl.

8. Compounds as claimed in claim 7 in which $R_3$ represents hydroxymethyl, hydroxyethyl, acyloxy $C_{1-2}$ alkyl, formyl, the group CH=NOH or methylsulphonylmethyl.

9. A process for the preparation of compounds as claimed in any of claims 1 to 8, which comprises:

a) cyclising a compound of formula (II)

$$R_1R_2NAlkQ(CH_2)_nX(CH_2)_m-\underset{\underset{V}{\|}}{C}-NHNH-\underset{\underset{Y}{\|}}{C}R_3 \qquad (II)$$

in which one of V and Y represents NH and the other represents oxygen, sulphur or NH or, when one of V and Y represents sulphur, an S-alkyl derivative of the tautomeric thiol; or

b) for the preparation of compounds in which $R_3$ is the group $-COR_4$, oxidising the corresponding hydroxyalkyl compound in which $R_3$ is $-CHR_4OH$; or

c) for the preparation of compounds in which $R_3$ is the group $-CR_4=NOH$, reacting the corresponding carbonyl compound in which $R_3$ is $-COR_4$ with hydroxylamine; or

d) for the preparation of compounds of formula (I) in which $R_3$ represents an acyloxyalkyl group, reacting a compound of formula (I) in which $R_3$ represents a hydroxyalkyl group with the carboxylic acid which corresponds to the acyl group or an activated derivative thereof;

and where the compound of formula (I) is produced in the form of a free base, optionally converting the free base into a salt.

10. A pharmaceutical composition comprising a compound as claimed in any of claims 1 to 8 and at least one inert pharmaceutically acceptable carrier or diluent, optionally together with at least one other active ingredient.

CLAIMS (AUSTRIA)

1. A process for the preparation of compounds of the general formula (I)

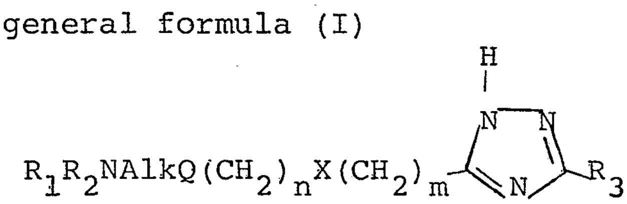

(I)

and physiologically acceptable salts, hydrates and bio-precursors thereof in which

$R_1$ represents hydrogen, $C_{1-10}$ alkyl, cycloalkyl, alkenyl, alkynyl, aralkyl, heteroaralkyl, trifluoroalkyl or $C_{1-10}$ alkyl substituted by hydroxy, alkoxy or dialkylamino; or $C_{1-6}$ alkyl substituted by cycloalkyl;

$R_2$ represents hydrogen or a $C_{1-4}$ alkyl group;

or $R_1$ and $R_2$ may together with the nitrogen atom to which they are attached form a 5-10 membered ring which may be saturated or may contain at least one double bond, may be unsubstituted or substituted by one or more $C_{1-3}$ alkyl groups or a hydroxy group and/or may contain another heteroatom selected from oxygen or sulphur;

Alk represents a straight or branched $C_{1-6}$ alkylene chain;

Q represents a benzene ring in which incorporation into the rest of the molecule is through bonds at the 1- and 3-positions or 1- and 4-positions;

X represents oxygen, sulphur or a methylene group;

n represents zero, 1 or 2;

m represents 2, 3 or 4 with the proviso that n + m is at least 3;

$R_3$ represents alkenyl, alkoxy or alkyl substituted by hydroxy, acyloxy, alkoxy, amino, alkylamino, dialkylamino, aryl, heteroaryl or $C_{1-4}$ alkylsulphonyl; or $R_3$ represents $COR_4$ or $CR_4$=NOH where $R_4$ represents hydrogen or $C_{1-4}$ alkyl, which comprises

a) cyclising a compound of formula (II)

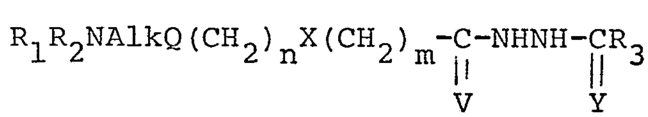

(II)

in which one of V and Y represents NH and the other represents oxygen, sulphur or NH or, when one of V and Y represents sulphur, an S-alkyl derivative of the tautomeric thiol; or

b) for the preparation of compounds in which $R_3$ is the group $-COR_4$, oxidising the corresponding hydroxyalkyl compound in which $R_3$ is $CHR_4OH$; or

c) for the preparation of compounds in which $R_3$ is the group $-CR_4=NOH$, reacting the corresponding carbonyl compound in which $R_3$ is $-COR_4$ with hydroxylamine; or

d) for the preparation of compounds of formula (I) in which $R_3$ represents an acyloxyalkyl group, reacting a compound of formula (I) in which $R_3$ represents a hydroxyalkyl group with the carboxylic acid which corresponds to the acyl group or an activated derivative thereof.

and where the compound of formula (I) is produced in the form of a free base, optionally converting the free base into a salt.

2. A process as claimed in claim 1 for the preparation of compounds in which Q is a benzene ring incorporated into the rest of the molecule through bonds at the 1- and 3-positions.

3. A process as claimed in claim 1 or 2 for the preparation of compounds in which X is oxygen, n is zero and m is 3 or 4.

4. A process as claimed in any of claims 1 to 3 for the preparation of compounds in which $R_1R_2N$ is a 5-7 membered ring.

5. A process as claimed in any of claims 1 to 4 for the preparation of compounds in which Alk is methylene.

6. A process as claimed in any of claims 1 to 5 for the preparation of compounds in which $R_3$ represents alkenyl, alkoxy, or alkyl substituted by hydroxy, acyloxy, alkoxy,

amino, alkylamino, dialkylamino, aryl or heteroaryl.

7. A process as claimed in any of claims 1 to 5 for the preparation of compounds in which $R_3$ is aralkyl, hydroxy $C_{1-4}$ alkyl, acyloxy $C_{1-4}$ alkyl, formyl, the group CH=NOH or methylsulphonylmethyl.

8. A process as claimed in claim 7 for the preparation of compounds in which $R_3$ represents hydroxymethyl, hydroxyethyl, acyloxy $C_{1-2}$ alkyl, formyl, the group CH=NOH or methylsulphonylmethyl.

0050407

European Patent Office

# EUROPEAN SEARCH REPORT

Application number
EP 81 30 3931

| Category | DOCUMENTS CONSIDERED TO BE RELEVANT<br>Citation of document with indication, where appropriate, of relevant passages | Relevant to claim | CLASSIFICATION OF THE APPLICATION (Int. Cl. 3) |
|---|---|---|---|
| | GB - A - 1 602 458 (SMITH KLINE)<br>* Whole document * | 1,9,10 | C 07 D 249/08<br>403/12<br>A 61 K 31/41//<br>C 07 D 295/08<br>C 07 C 119/18 |
| | -- | | |
| | GB - A - 1 431 589 (SMITH KLINE)<br>* Whole document * | 1,9,10 | |
| | -- | | |
| | GB - A - 1 398 426 (SMITH KLINE)<br>* Whole document * | 1,9,10 | |
| | ---- | | |

TECHNICAL FIELDS SEARCHED (Int.Cl. 3)

C 07 D 249/08
403/12
A 61 K 31/41

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 07-12-1981 | CREMERS |